# EUROPÄISCHE PATENTANMELDUNG

(11) **EP 2 910 546 A1**
(43) Veröffentlichungstag der Anmeldung: **26.08.2015**
(21) Anmeldenummer: 15154262.8
(22) Anmeldetag: 09.02.2015
(51) Int. Cl.: C07D 209/08, C07D 209/86

(54) **Verfahren zur photokatalytischen akzeptorfreien Dehydrierung von Hydrocarbazolen und Hydroindolen**

(30) Priorität: 25.02.2014 DE 102014203345
(71) Anmelder: Evonik Degussa GmbH, 45128 Essen (DE)
(72) Erfinder: Franke, Robert, 45772 Marl (DE); Julis, Jennifer, 40479 Düsseldorf (DE); Fridag, Dirk, 45721 Haltern am See (DE); Dyballa, Katrin Marie, 45657 Recklinghausen (DE); Weding, Nico, 45657 Recklinghausen (DE); Dutta Chowdhury, Abhishek, 743222 West Bengal (IN); Beller, Matthias, 18211 Nienhagen (DE); Jackstell, Ralf, 27478 Cuxhaven Altenwalde (DE); Fleischer, Ivana, 93057 Regensburg (DE); Percia, Beatrice, 624802 Vadamadurai, TamilNadu (IN)

(57) **Zusammenfassung**

Die Erfindung betrifft ein Verfahren zur photokatalytischen akzeptorfreien Dehydrierung von Hydrocarbazolen und Hydroindolen bei dem ein Hydrocarbazol oder Hydroindol in Gegenwart einer Rhodiumkomplexverbindung, die organische Phosphor(III)-Verbindungen als Liganden enthält, als Katalysator bestrahlt wird.

## Beschreibung

Die Erfindung betrifft ein Verfahren zur photokatalytischen akzeptorfreien Dehydrierung von Hydrocarbazolen und Hydroindolen.

Die akzeptorfreie Dehydrierung wird aufgrund der Einfachheit und Atomökonomie als ein idealer Prozess betrachtet. Solch eine atomökonomische und akzeptorfreie Dehydrierung kann unter photokatalytischen Bedingungen erreicht werden. Jedoch ist diese Reaktion durch lange Reaktionszeiten, Katalysatordeaktivierung und geringere Reaktivität im Vergleich zur Dehydrierung/Transferhydrierungsreaktion limitiert. Von Nomura, K.; Saito, Y. J. Chem. Soc., Chem. Commun. 1988, 161-162 ist bekannt, dass der Katalysator Rh(PMe₃)₂(CO)Cl aktiv für eine akzeptorfreie photokatalytische Dehydrierung und in der thermochemischen Transferhydrierung ist, hier aber nur unter Wasserstoffdruck, was dessen Anwendbarkeit zur Synthese von Alkenen einschränkt.

Um Dehydrierung bei relativ niedriger Temperatur unter homogenen Bedingungen zu realisieren, werden normalerweise Opferolefine (Akzeptoren) eingesetzt, um die hohe Endothermie zu überwinden. Allgemein ist in der Literatur hierzu eine effiziente Alkantransferhydrierung nur mit einem großen Überschuss eines Opferolefins (bis zu 20-fachem Überschuss) erreicht worden, was eine potentielle sinnvolle Anwendbarkeit einschränkt. Auch sind die technisch relevanten Turnoverzahlen (TON) dieser Methoden normalerweise mit ca. 1000 bei vernünftigen Reaktionszeiten limitiert. Turnoverzahlen (TON) >1000 werden erst bei langen Reaktionszeiten von mehreren Tagen erreicht. Obwohl es verschiedene kontinuierliche Anstrengungen zu den homogenen katalytischen Dehydrierungen in den letzten 30 Jahren gab, ist immer noch ein erheblicher Bedarf für signifikante Verbesserungen gegeben, speziell für die akzeptorfreie atomökonomische Dehydrierung.

Derzeit erfolgreiche Dehydrierungen hängen hauptsächlich vom Verhalten verschiedener spezieller anspruchsvoller Pincerliganden und deren thermischen Stabilität (auch ihrer Metallkomplexe) ab. Um die hohe Endothermie der Alkandehydrierung zu überwinden wurden normalerweise hohe Reaktionstemperaturen von bis zu 250 °C oder lange Reaktionszeiten von bis zu 3 Tagen angewendet. Deshalb ist die Reaktivität streng durch die thermische Stabilität des Katalysators bestimmt. Ein weiteres Problem stellt die Inhibierung der Reaktion durch das Olefin dar, welches entweder als Opferolefin oder als Produkt präsent ist, speziell bei längeren Reaktionszeiten.

Die Aufgabe bestand darin ein Dehydrierungsverfahren bereitzustellen, welches die im Stand der Technik genannten Nachteile nicht aufweist.

Gelöst wird die Aufgabe durch ein Verfahren nach Anspruch 1.

Verfahren zur photokatalytischen akzeptorfreien Dehydrierung von Hydrocarbazolen und Hydroindolen dadurch gekennzeichnet, dass ein Hydrocarbazol oder Hydroindol in Gegenwart einer Rhodiumkomplexverbindung, die organische Phosphor(III)-Verbindungen als Liganden enthält, als Katalysator bestrahlt wird.

In einer Variante des Verfahrens ist das Hydrocarbazol Dodecahydro-N-ethylcarbazol oder Dodecahydro-N-methylcarbazol.

In einer Variante des Verfahrens erfolgt die Dehydrierung unter Zusatz einer Lewis Base.

In einer Variante des Verfahrens ist die Lewis Base ein organisches Amin.

In einer Variante des Verfahrens ist die Lewis Base ein heterozyklisches Amin.

In einer Variante des Verfahrens ist die Lewis Base ein Bipyridin.

In einer Variante des Verfahrens wird als Lewis Base 2,2-Bipyridin oder 4,4-Bipyridin verwendet.

In einer Variante des Verfahrens wird als Lewis Base Bathocuproin oder Phenanthrolin verwendet.

In einer Variante des Verfahrens wird die Reaktion in Gegenwart von CO₂ durchgeführt.

Es wurde des Weiteren gefunden, dass in Gegenwart von CO₂ die Reaktionen zur Dehydrierung besser verlaufen mit höheren Ausbeuten bei längerer Reaktionsführung.

In einer Variante des Verfahrens erfolgt die Bestrahlung mit Licht einer Wellenlänge von 320 nm bis 500 nm.

Auch der Einfluss der Wellenlänge der verwendeten Lichtquelle wurde untersucht und es wurde gefunden, dass eine Bestrahlung bevorzugt mit Licht einer Wellenlänge von λ = 320 nm bis 500 nm erfolgt.

In einer Variante des Verfahrens wird die Dehydrierung bei einer Temperatur von 45 °C bis 120 °C durchgeführt.

Die erfindungsgemäße Dehydrierung findet weiterhin besonders optimal bei einer Temperatur von vorzugsweise 45 °C bis 120 °C statt. Eine Temperatur von 80 °C bis 95 °C ist besonders bevorzugt. Die besten Ausbeuten werden bei einer Reaktionstemperatur von 85 °C bis 90 °C erzielt.

In einer Variante des Verfahrens wird ein Rhodiumkomplex der allgemeinen Formel (I) eingesetzt:

Rh(L¹)₂(CO)X (I),

worin L¹ = P(C₁-C₅Alkyl)₃ ist,
und X für ein Anion steht ausgewählt aus: Chlorid, Bromid, Acetat.

In einer Variante des Verfahrens ist L¹ = PMe₃ oder P*t*Bu₃.

In einer Variante des Verfahrens steht X für Chlorid.

In einer Variante des Verfahrens wird ein Rhodiumkomplex der allgemeinen Formel (**II**) eingesetzt:

Rh₂(L²)₂(CO)₂(X)₂ (II),

worin L² = P(PH)₂(CH₂)ₙ(Ph₂)P ist mit n = 1 bis 3,
und X für ein Anion steht ausgewählt aus: Chlorid, Bromid, Acetat.

In einer Variante des Verfahrens ist L² = P(PH)₂(CH₂)(Ph₂)P.

In einer Variante des Verfahrens steht X für Chlorid.

In einer Variante des Verfahrens wird der gebildete Wasserstoff aus dem Reaktionsgemisch entfernt.

Um eine noch effektivere Reaktion durchzuführen, kann der gebildete Wasserstoff vorteilhafter Weise aus der Reaktionslösung entfernt werden. Das erfolgt z.B. durch eine hohe Rührgeschwindigkeit und konstantem Gasstrom (z.B. Argon). Durch diese Reaktionsführung können die erzielten TON weiter erhöht werden und sind somit wesentlich höher als in der Literatur beschriebene Systeme.

In einer Variante des Verfahrens erfolgt die Bestrahlung durch eine Glasscheibe hindurch erfolgt.

Darüber hinaus ist die Verwendung eines Glasreaktors oder eines Metallreaktors, der Glas aufweist, im erfindungsgemäßen Verfahren besonders effektiv. Einwandige Glasreaktoren, vorzugsweise mit einer Wandstärke von 1,0 bis 3,0 mm, sind besonders geeignet. Interessanterweise findet kaum eine Reaktion statt, wenn man lediglich einen geeigneten Metallreaktor mit Lichteingang verwendet. Überraschend hat die Gegenwart von Glas einen bemerkenswerten Einfluss auf die erfindungsgemäße Reaktion, wobei neben dem Material auch die Wandstärke des verwendeten Glasgefäßes bedeutsam sein kann. So kann die Reaktion zwar mit Glasgefäßen unterschiedlicher Wandstärke durchgeführt werden, eine Wandstärke von 1,2 mm bis 1,8 mm hat sich als besonders optimal erwiesen.

In einer Variante des Verfahrens weist die Glasscheibe eine Dicke von 1,2 mm bis 3,0 mm auf.

Vorteilhafterweise werden deshalb dünnwandige Glasgefäße im erfindungsgemäßen Verfahren eingesetzt, da die Transmission in diesen erheblich größer ist und deshalb mehr Energie zur Alkandehydrierung zur Verfügung steht. Dieser starke Einfluss der Anwesenheit von Glas und der Wandstärke der Reaktionsgefäße wurde bis jetzt noch nicht in photokatalytischen Reaktionen nachgewiesen.

In einer Variante des Verfahrens werden einwandige Glasreaktoren eingesetzt.

Die Bestrahlung erfolgt bevorzugt mit Licht, vorzugsweise mit einer Wellenlänge von λ = 320 nm bis 500 nm. Die Dehydrierung erfolgt weiterhin besonders optimal bei einer Temperatur von vorzugsweise 45 °C bis 120 °C, vorzugsweise bei einer Temperatur von 80 °C bis 95 °C. Insbesondere ist eine Reaktionstemperatur von 85 °C bis 90 °C effektiv. Weiterhin ist die Verwendung eines Glasreaktors oder eines Metallreaktors, der Glas aufweist, im erfindungsgemäßen Verfahren besonders effektiv. Einwandige Glasreaktoren, vorzugsweise mit einer Glasdicke von 1,0 bis 3,0 mm, sind besonders geeignet. Der verwendete Rhodiumkomplex ist vorzugsweise einer der allgemeinen Formel (I), der oben genannt ist, insbesondere mit L¹ = PMe₃ oder PBu₃ und X = Cl. In der Regel ist der bevorzugte Katalysator Rh(PMe₃)₂(CO)Cl.

Das Verfahren kann zur photokatalytischen akzeptorfreien Dehydrierung von Hydrocarbazolen verwendet werden, wobei ein Hydrocarbazol in Gegenwart einer Rhodiumkomplexverbindung, die organische Phosphor(III)-Verbindungen als Liganden enthält, als Katalysator bestrahlt wird. Vorzugsweise ist das Hydrocarbazol Dodecahydro-N-ethylcarbazol oder Dodecahydro-N-methylcarbazol.

In einer anderen erfindungsgemäßen Variante kann das Verfahren auch zur photokatalytischen akzeptorfreien Dehydrierung von Hydroindolin verwendet werden, wobei ein Hydroindolin in Gegenwart einer Rhodiumkomplexverbindung, die organische Phosphor(III)-Verbindungen als Liganden enthält, als Katalysator bestrahlt wird. Vorzugsweise ist das Hydroindolin Indolin.

Die Dehydrierung von Hydrocarbazolen wie Dodecahydro-N-ethylcarbazol kann unter lösungsmittelfreien Bedingungen photokatalytisch effektiv realisiert werden. In der Literatur wird eine vollständige Hydrierung von N-ethylcarbazol zu Dodecahydro-N-ethylcarbazol als chemischer Wasserstoffspeicher mit 5% Ru/Al₂O₃ nach zwei Tagen erreicht [Morawa Eblagon, K.; Tam, K.; Kerry Yu, K. M.; Zhao, S.-L.; Gong, X.-Q.; He, H.;Ye, L.;Wang, L.-C.;Ramirez-Cuesta, A. J.;Tsang, S. C. J. Phys. Chem. C 2010, 114, 9720-9730]. Die erwünschte Dehydrierung erfolgt in der Literatur nur bei sehr hohen Temperaturen heterogenkatalysiert von 200 °C während im erfindungsgemäßen photokatalytischen Verfahren bei bevorzugten Temperaturen um 90 °C sowohl vollständig und partiell dehydrierte Carbazole erzielt werden. So stellt das Verfahren eine geeignete homogen katalytische Methode zur milden Dehydrierung von potentiellen chemischen Wasserstoffspeichern dar.

Im Folgenden wird die Erfindung an Ausführungsbeispielen näher erläutert.

### Ausführungsbeispiele

**Allgemeine Reaktionsbedingungen:** Alle synthetischen Operationen wurden unter Argon in getrockneten Duranborosilicatglasgefäßen mit geeigneten Schlenktechniken durchgeführt. Der Katalysator Rh(PMe₃)₂(CO)Cl wurde analog einer Literaturvorschrift hergestellt. [Bridgewater, J. S.; Netzel, T. L.; Schoonover, J. R.; Massick, S. M.; Ford, P. C. Inorg. Chem. 2001, 40, 1466-1476]. Die Produkte wurden gegen eine Vergleichsprobe analysiert und die Ausbeute mittels Gaschromatographie (Agilent 6890N network GC System mit einer (60m×250µm×0.25µm) DB Wax Säule und Isooctan als internen Standard (0,2 ml, 1,2 mmol) nach einer Verdünnung mit Aceton. Die Responsfaktoren jedes Produktes wurden mittels 'Multiple Point Internal Standard GC Quantitation Method' gegen Isooctan bestimmt. Für alle Analysen wurden folgende Bedingungen gewählt: N₂ als Trägergas, Einlasstemperatur: 250 °C, Einlassdruck: 104.4 KPa, Injektionsvolumen: 1,0 µl, Splitverhältnis: 100:1, Splitflow: 80,0 ml/min, Flussrate: 0,8 ml/min bis 20 min die dann auf 2,8 ml/min 1.0 ml/min² erhöht wurde, Temperatur: 35 °C bis 20 min, anschließend erhöht auf 40 °C/min bis 200 °C und dann 15 min bei 200 °C gehalten. Detektortemperatur: 250 °C, Wasserstofffluss: 30 ml/min, Luft: 300 ml/min, Stickstroffstrom: 25 ml/min.

Allgemeine Arbeitsvorschrift 1: Das entsprechende Glasgefäß versehen mit einem Rückflusskühler und Magnetrührer wird unter Argon mit 0,004 mmol des Katalysators Rh(PMe₃)₂(CO)Cl und 0,02 mmol des entsprechenden Additives befüllt. Sehr genaues Arbeiten unter Argon als Schutzgas ist notwendig, da der Katalysator sehr leicht in Gegenwart von Luftsauerstoff und Licht deaktiviert wird. Es werden anschließend 30 mmol Substrat zugesetzt und ein Argonstrom angelegt. Die Rührgeschwindigkeit wird auf 1000 min⁻¹ eingestellt und das Glasgefäß mit Aluminiumfolie umschlossen. Dann wird die verwendete Lichtquelle Lumatec Superlite 400, die Licht über einen Wellenlängenbereich von 320 nm bis 500 nm ausstrahlt, angeschaltet. Nach der Reaktion wird die Lichtquelle ausgeschaltet, die Reaktionslösung abgekühlt und die Ausbeute mittels Gaschromatografie und Isooctan als internem Standard bestimmt. Die Turnoverzahlen (TON) in den Tabellen werden mit [mmol Produkt]/[mmol Katalysator] berechnet.

Allgemeine Arbeitsvorschrift 2: Das entsprechende Glasgefäß versehen mit einem Rückflusskühler und Magnetrührer wird unter Argon mit 0,004 mmol des Katalysators Rh(PMe₃)₂(CO)Cl und 0,02 mmol des entsprechenden Additives befüllt. Sehr genaues Arbeiten unter Argon als Schutzgas ist notwendig, da der Katalysator sehr leicht in Gegenwart von Luftsauerstoff und Licht deaktiviert wird. Es werden anschließend 30 mmol Substrat zugesetzt und ein Argonstrom angelegt. Die Rührgeschwindigkeit wird auf 1000 min⁻¹ eingestellt und das Glasgefäß mit Aluminiumfolie umschlossen. Es wird mittels einer Metallkapillare ein CO₂-Strom durch die Lösung geleitet. Dann wird die verwendete Lichtquelle Lumatec Superlite 400 angeschaltet. Nach der Reaktion wird die Lichtquelle ausgeschaltet, der CO₂-Strom beendet, die Reaktionslösung abgekühlt und die Ausbeute mittels Gaschromatografie und Isooctan als internem Standard bestimmt. Die Turnoverzahlen (TON) in den Tabellen werden mit [mmol Produkt]/[mmol Katalysator] berechnet.

### Beispiel

### Dehydrierung von Dodecahydro-N-ethylcarbazol (H₁₂NEC)

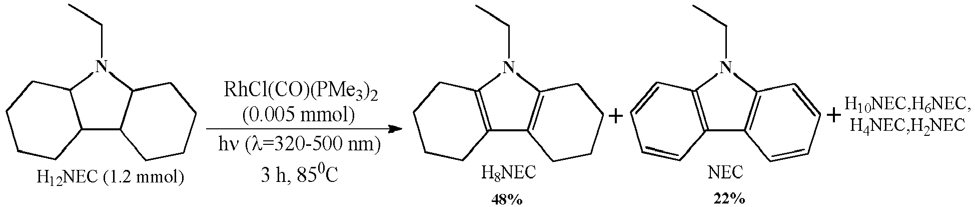

Ein Schlenkgefäß mit einer Wandstärke von 1,2 mm versehen mit einem Rückflusskühler und Magnetrührer wird mit 1,6 mg Rh(PMe₃)₂(CO)Cl (0,005 mmol) bestückt. Anschließend wird das Reaktionsgefäß dreimal evakuiert und mit Argon befüllt, um inerte Bedingungen zu realisieren. Es werden anschließend 250 mg Dodecahydro-N-ethylcarbazol (1,2 mmol) zugegeben. Es wird ein Argonstrom angelegt, um gebildeten Wasserstoff zu entfernen. Der Reaktor wird mit Aluminiumfolie umschlossen und die Lichtquelle mit einer Wellenlänge 320-500 nm Wellenlänge (Lumatec Superlite 400) angeschaltet. Es wird drei Stunden bei 1000 min⁻¹ gerührt. Nach der Reaktion wird die Lichtquelle ausgeschaltet und die Reaktionslösung abgekühlt. Die Ausbeute wird mittels Gaschromatografie und Isooctan als internem Standard bestimmt.

**Tabelle**

| Substrat | Zeit (h) | Ausbeute (%) | TON | TOF (h⁻¹) |
|---|---|---|---|---|
| H₁₂NEC | 3 | 80 | 188 | 63 |

Wie aus der Tabelle ersichtlich ist konnte das Produkt innerhalb von 3 Stunden mit einer sehr guten Ausbeute erhalten werden.

## Patentansprüche

1. Verfahren zur photokatalytischen akzeptorfreien Dehydrierung von Hydrocarbazolen und Hydroindolen **dadurch gekennzeichnet, dass** ein Hydrocarbazol oder Hydroindol in Gegenwart einer Rhodiumkomplexverbindung, die organische Phosphor(III)-Verbindungen als Liganden enthält, als Katalysator bestrahlt wird.

2. Verfahren nach Anspruch 1,
**dadurch gekennzeichnet, dass** das Hydrocarbazol Dodecahydro-N-ethylcarbazol oder Dodecahydro-N-methylcarbazol ist.

3. Verfahren nach Anspruch 1 oder 2,
**dadurch gekennzeichnet, dass** die Dehydrierung unter Zusatz einer Lewis Base erfolgt.

4. Verfahren nach Anspruch 3,
wobei die Lewis Base ein organisches Amin ist

5. Verfahren nach einem der Ansprüche 3 oder 4,
wobei die Lewis Base ein heterozyklisches Amin ist.

6. Verfahren nach einem der Ansprüche 3 bis 5,
wobei die Lewis Base ein Bipyridin ist.

7. Verfahren nach einem der Ansprüche 3 bis 6,
wobei als Lewis Base 2,2-Bipyridin oder 4,4-Bipyridin verwendet wird.

8. Verfahren nach einem der Ansprüche 3 bis 7,
wobei als Lewis Base Bathocuproin oder Phenanthrolin verwendet wird.

9. Verfahren nach einem der Ansprüche 1 bis 8,
**dadurch gekennzeichnet, dass** die Reaktion in Gegenwart von CO₂ durchgeführt wird.

10. Verfahren nach einem der Ansprüche 1 bis 9,
**dadurch gekennzeichnet, dass** die Bestrahlung mit Licht einer Wellenlänge von 320 nm bis 500 nm erfolgt.

11. Verfahren nach einem der Ansprüche 1 bis 10,
**dadurch gekennzeichnet, dass** die Dehydrierung bei einer Temperatur von 45 °C bis 120 °C durchgeführt wird.

12. Verfahren nach einem der Ansprüche 1 bis 11,
**dadurch gekennzeichnet, dass** ein Rhodiumkomplex der allgemeinen Formel (I) eingesetzt wird:
Rh(L¹)₂(CO)X (I),
worin L¹ = P(C₁-C₅Alkyl)₃ ist,
und X für ein Anion steht ausgewählt aus: Chlorid, Bromid, Acetat.

13. Verfahren nach Anspruch 12,
**dadurch gekennzeichnet, dass** L¹ = PMe₃ oder P*t*Bu₃.

14. Verfahren nach einem der Ansprüche 12 oder 13,
**dadurch gekennzeichnet, dass** X für Chlorid steht.

15. Verfahren nach einem der Ansprüche 1 bis 11,
**dadurch gekennzeichnet, dass** ein Rhodiumkomplex der allgemeinen Formel **(II)** eingesetzt wird:
Rh₂(L²)₂(CO)₂(X)₂ (II),
worin L² = P(PH)₂(CH₂)ₙ(Ph₂)P ist mit n = 1 bis 3,
und X für ein Anion steht ausgewählt aus: Chlorid, Bromid, Acetat.

16. Verfahren nach Anspruch 15,
**dadurch gekennzeichnet, dass** L² = P(PH)₂(CH₂)(Ph₂)P.

17. Verfahren nach einem der Ansprüche 15 oder 16,
**dadurch gekennzeichnet, dass** X für Chlorid steht.

18. Verfahren nach einem der Ansprüche 1 bis 17,
**dadurch gekennzeichnet, dass** der gebildete Wasserstoff aus dem Reaktionsgemisch entfernt wird.

## Geänderte Patentansprüche

### Geänderte Patentansprüche gemäss Regel 137(2) EPÜ.

1. Verfahren zur photokatalytischen akzeptorfreien Dehydrierung von Hydrocarbazolen und Hydroindolen **dadurch gekennzeichnet, dass** ein Hydrocarbazol oder Hydroindol in Gegenwart einer Rhodiumkomplexverbindung, die organische Phosphor(III)-Verbindungen als Liganden enthält, als Katalysator bestrahlt wird,
wobei ein Rhodiumkomplex der allgemeinen Formel (I) eingesetzt wird:
Rh(L¹)₂(CO)X **(I),**
worin L¹ = P(C₁-C₅Alkyl)₃ ist,
und X für ein Anion steht ausgewählt aus: Chlorid, Bromid, Acetat;
oder
ein Rhodiumkomplex der allgemeinen Formel **(II)** eingesetzt wird:
Rh₂(L²)₂(CO)₂(X)₂ **(II),**
worin L²=P(PH)₂(CH₂)ₙ(Ph₂)P ist mit n = 1 bis 3,
und X für ein Anion steht ausgewählt aus: Chlorid, Bromid, Acetat.

2. Verfahren nach Anspruch 1,
**dadurch gekennzeichnet, dass** das Hydrocarbazol Dodecahydro-N-ethylcarbazol oder Dodecahydro-N-methylcarbazol ist.

3. Verfahren nach Anspruch 1 oder 2,
**dadurch gekennzeichnet, dass** die Dehydrierung unter Zusatz einer Lewis Base erfolgt.

4. Verfahren nach Anspruch 3,
wobei die Lewis Base ein organisches Amin ist

5. Verfahren nach einem der Ansprüche 3 oder 4,
wobei die Lewis Base ein heterozyklisches Amin ist.

6. Verfahren nach einem der Ansprüche 3 bis 5,
wobei die Lewis Base ein Bipyridin ist.

7. Verfahren nach einem der Ansprüche 3 bis 6,
wobei als Lewis Base 2,2-Bipyridin oder 4,4-Bipyridin verwendet wird.

8. Verfahren nach einem der Ansprüche 3 bis 7,
wobei als Lewis Base Bathocuproin oder Phenanthrolin verwendet wird.

9. Verfahren nach einem der Ansprüche 1 bis 8,
**dadurch gekennzeichnet, dass** die Reaktion in Gegenwart von CO₂ durchgeführt wird.

10. Verfahren nach einem der Ansprüche 1 bis 9,
**dadurch gekennzeichnet, dass** die Bestrahlung mit Licht einer Wellenlänge von 320 nm bis 500 nm erfolgt.

11. Verfahren nach einem der Ansprüche 1 bis 10,
**dadurch gekennzeichnet, dass** die Dehydrierung bei einer Temperatur von 45 °C bis 120 °C durchgeführt wird.

12. Verfahren nach einem der Ansprüche 1 bis 11,
**dadurch gekennzeichnet, dass** L¹=PMe₃ oder P*t*Bu₃.

13. Verfahren nach einem der Ansprüche 1 bis 12,
**dadurch gekennzeichnet, dass** X in **(I)** für Chlorid steht.

14. Verfahren nach einem der Ansprüche 1 bis 13,
**dadurch gekennzeichnet, dass** L²=P(PH)₂(CH₂)(Ph₂)P.

15. Verfahren nach einem der Ansprüche 1 bis 14,
**dadurch gekennzeichnet, dass** X in **(II)** für Chlorid steht.

16. Verfahren nach einem der Ansprüche 1 bis 15,
**dadurch gekennzeichnet, dass** der gebildete Wasserstoff aus dem Reaktionsgemisch entfernt wird.
